Europäisches Patentamt

European Patent Office

Office européen des brevets

(19)

(11) Numéro de publication: **0 107 578**
**A2**

## (12) DEMANDE DE BREVET EUROPEEN

(21) Numéro de dépôt: **83401998.6**

(22) Date de dépôt: **13.10.83**

(51) Int. Cl.³: **A 61 B 5/14**

(30) Priorité: **21.10.82 FR 8217633**

(43) Date de publication de la demande: **02.05.84**
**Bulletin 84/18**

(84) Etats contractants désignés: **AT BE CH DE GB IT LI LU NL SE**

(71) Demandeur: **LE MATERIEL BIOMEDICAL Société à Responsabilité Limitée dite:, 4 rue de Presbourg, F-75116 Paris (FR)**

(72) Inventeur: **Lachenal, Jean-Michel, 25 avenue de la Paix, F-94260 Fresnes (FR)**

(74) Mandataire: **Bonnetat, Christian et al, Cabinet PROPI Conseils 23 rue de Léningrad, F-75008 Paris (FR)**

(54) **Système de prélèvement de liquide physiologique, tel que du sang.**

(57) Système de prélèvement de liquide physiologique, tel que du sang, comportant des moyens d'aspiration (2) et au moins un récipient (3) susceptible d'être relié, par une des extrémités, de manière étanche amovible aux-dits moyens d'aspiration (2), ledit récipient (3) comportant une cloison transversale (8), disposée au voisinage de ladite extrémité reliée aux moyens d'aspiration (2) ou obturant cette extrémité et percée d'un orifice (9), tandis qu'un organe de prélèvement (11) dudit liquide est relié de façon amovible à l'autre extrémité du récipient (3), opposée aux moyens d'aspiration (2).

Selon l'invention, ce système est caractérisé en ce que la partie (3a) dudit récipient (3) comprise entre la cloison (8) et l'extrémité reliée audit organe de prélèvement (11), est conformée en cylindre pour un bouchon glissant (12), disposé dans ladite partie (3a) et formant piston libre à l'intérieur de celle-ci.

Système de prélèvement de liquide physiologique, tel que du sang.

La présente invention concerne un système de prélèvement de liquide physiologique, tel que du sang, et plus particulièrement un sytème permettant le prélèvement d'échantillons de faible volume.

De façon connue, le prélèvement de sang, notamment en vue de la constitution d'échantillons, est effectué à l'aide d'une seringue à aiguille, la traction du piston entraînant l'aspiration du sang dans le corps de la seringue.

Dans le cas où la quantité de sang prélevé est destinée à subir des opérations d'analyse ou de test, on doit, de façon connue, transférer le volume de sang contenu dans le corps de la seringue dans un ou plusieurs récipients selon le nombre d'opérations à effectuer.

Ce transfert entraîne des risques de contamination (par exemple en cas d'hépatite) de l'opérateur par le sang éventuellement infecté avec les conséquences graves que cette situation implique. Ce risque de contamination est d'autant plus élevé que le nombre d'analyses, donc de récipients différents, est plus grand.

En outre, ce transfert de sang prélevé, du corps de la seringue dans le ou les récipients, est susceptible de donner lieu à des erreurs ou à des difficultés de manipulations, notamment dans le cas de faibles volumes.

De plus, grâce aux perfectionnements des techniques et du matériel d'analyse sanguine, on peut maintenant obtenir des analyses et tests satisfaisants à partir de très faibles quantités de sang (quelques mm³). Dans de nombreux cas, on peut prélever suffisamment de sang par la méthode capillaire (bout de doigt, oreille, etc...), qui met en oeuvre des tubes capillaires. Le contenu de ces tubes, après prélèvement, doit être transféré dans un autre récipient à des fins d'analyse. On conçoit qu'il est très difficile de prélever et transférer de façon précise de petits volumes de sang avec une seringue classique, et que cela est même impossible avec de très petits volumes.

Afin de remédier à ces inconvénients, on a déjà décrit dans la demande de brevet européen EP-A1-0 055 657 un système de prélèvement de sang (aussi bien capillaire que veineux) de mise en oeuvre simple, destiné à obtenir un volume de sang faible et correspondant exactement à la quantité désirée, les risques de contamination étant limités, du fait que le sang prélevé est introduit dans le récipient qui est directement utilisé pour réaliser les analyses et les tests envisagés.

Ce système de prélèvement de liquide physiologique, tel que du sang, comporte des moyens d'aspiration manuelle ou mécanique, par exemple une seringue, pourvus d'un cylindre et d'une aiguille, et est remarquable en ce qu'il comporte au moins un récipient tubulaire de faible diamètre susceptible d'être maintenu de façon amovible dans le prolongement du cylindre, du côté de l'aiguille, par des moyens de retenue portés par ledit cylindre, de manière que le fond dudit récipient soit traversé de façon étanche par ladite aiguille, ledit récipient étant pourvu d'un capuchon obturant son extrémité opposée à ladite aiguille et pourvu d'un organe de prélèvement dudit liquide.

On voit que, dans ce système, ladite aiguille sert de connexion entre les moyens d'aspiration et le récipient lorsque celui-ci est maintenu par les moyens de retenue et que l'aiguille traverse le fond dudit récipient, ce qui permet aux moyens d'aspiration d'aspirer l'air contenu dans le récipient et de forcer le liquide amené par l'organe de prélèvement à pénétrer dans ledit récipient, malgré le faible diamètre de celui-ci.

Ainsi, grâce à ce système, on évite le risque de contamination puisque le sang prélevé passe directement du patient dans le récipient qui sera utilisé pour effectuer les analyses ou tests nécessaires.

Lors de l'aspiration du sang par les moyens d'aspiration dans le cylindre, il est bien entendu nécessaire que le fond du récipient soit traversé par l'aiguille desdits moyens d'aspiration de manière étanche. Le fond doit en outre être étanche, lorsque ce dernier est désolidarisé des moyens d'aspiration pour effectuer les analyses ; autrement dit le sang contenu ne doit pas pouvoir sortir dudit récipient.

A cet effet, le fond du récipient comporte une cloison, en retrait de l'extrémité inférieure dudit récipient, qui est pourvue d'une lumière de diamètre suffisamment grand pour permettre le passage de l'aiguille des moyens d'aspiration et contre laquelle s'applique une membrane souple en un matériau élastique, tel que du caoutchouc, disposée du côté extérieur de la cloison et destinée à être percée par ladite aiguille, ladite membrane présentant une élasticité suffisante pour permettre, après enlèvement de l'aiguille, l'obturation de l'ouverture créée par l'enfoncement de ladite aiguille.

On obtient ainsi l'étanchéité aussi bien lorsque l'aiguille des moyens d'aspiration traverse la membrane, que lorsque ladite aiguille est retirée, lors de la désolidarisation du récipient et de ces moyens d'aspiration.

Cependant, ce système décrit dans le document EP-A1-0 055 657 s'est révélé d'un usage délicat. En effet, lors de l'actionnement des moyens d'aspiration mettant en dépression le récipient tubulaire, on s'est aperçu que ces moyens d'aspiration non seulement aspirent l'air du récipient tubulaire, permettant ainsi à celui-ci de se remplir de sang, mais encore le sang pénètre dans ledit récipient. Ceux-ci sont alors souillés et ne peuvent plus être utilisés pour un autre prélèvement. En cas d'actionnement brusque desdits moyens d'aspiration, il arrive même que, à la manière d'un arc électrique, il se forme un écoulement sanguin, direct et continu entre l'organe de prélèvement du capuchon monté sur le récipient et l'aiguille des moyens d'aspiration. La presque totalité du sang prélevé sur le donneur passe alors directement dans les moyens d'aspiration.

La présente invention a pour objet de remédier à cet inconvénient et de permettre le prélèvement direct de faibles volumes de sang.

A cette fin, selon la présente invention, le système de prélèvement de liquide physiologique, tel que du sang, comportant des moyens d'aspiration, par exemple du type seringue, et au moins un récipient susceptible d'être relié, par une de ses extrémités, de manière étanche et amovible auxdits moyens d'aspiration, ledit récipient comportant une cloison transversale, disposée au voisinage de ladite extrémité reliée aux moyens d'aspiration ou obturant cette extrémité et percée d'un orifice, tandis qu'un organe de prélèvement dudit liquide est relié de façon amovible à l'autre extrémité dudit récipient, est caractérisé en ce que la partie dudit récipient comprise entre ladite cloison et l'extrémité reliée audit organe de prélèvement est conformée en cylindre pour un bouchon glissant, disposé dans ladite partie et formant piston libre à l'intérieur de celle-ci.

Ainsi, ledit piston libre étant initialement disposé du côté de l'extrémité à laquelle est relié l'organe de prélèvement, lorsque l'on actionne lesdits moyens d'aspiration, ce piston libre recule vers la cloison interne car il est aspiré par ceux-ci à travers ledit orifice. En reculant, le piston libre aspire le liquide amené par l'organe de prélèvement et le récipient se remplit. Pendant le remplissage, le piston fait joint d'étanchéité par rapport à la paroi interne du récipient et le liquide ne peut pas passer jusqu'aux moyens d'aspiration. En fin d'aspiration, le piston libre se trouve appliqué contre la cloison interne (du côté de celle-ci opposé aux moyens d'aspiration) et obture de façon étanche l'orifice de ladite cloison.

Dans le cas où, de façon connue, lesdits moyens d'aspiration sont constitués par une seringue, il est avantageux que ledit récipient soit emboîtable sur ladite seringue sans l'intermédiaire d'une aiguille.
De plus, on voit que le volume de la partie du récipient comprise entre la cloison et l'extrémité du récipient à laquelle est rapporté ledit organe de prélèvement peut être très inférieur au volume intérieur du cylindre de la seringue. Ainsi, le récipient peut être de petite taille pour être adapté aux contraintes des méthodes de tests modernes, la seringue servant d'organe de manipulation dudit récipient pendant le prélèvement de sang.

De préférence, le bouchon glissant est formé par un bloc d'élastomère.

La figure unique du dessin annexé fera bien comprendre comment l'invention peut être réalisée.

Cette figure unique est une vue en coupe longitudiale d'un exemple de réalisation du système selon l'invention.

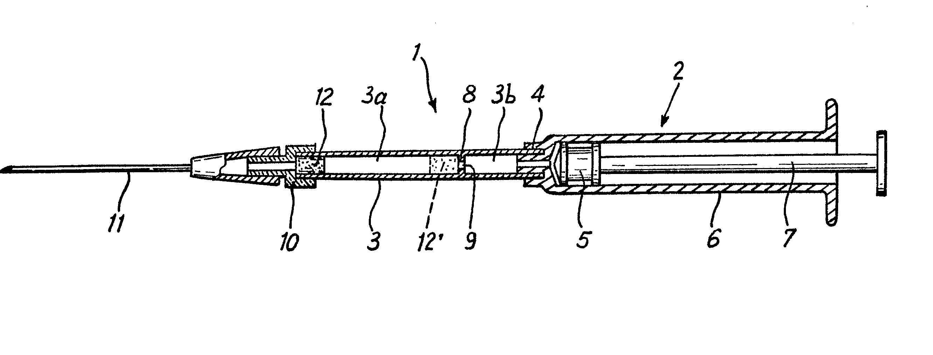

L'exemple de réalisation 1 du système selon l'invention pour le prélèvement de sang, montré sur la figure 1, comporte une seringue 2 et un récipient tubulaire 3 solidarisé de cette dernière par des moyens d'emboîtement 4 de type connu. La seringue 2 est composée de manière connue d'un piston 5, coulissant dans le cylindre 6 et solidaire d'une tige de manoeuvre 7. On remarquera que la mise en oeuvre de l'invention ne nécessite pas que la seringue 2 soit pourvue d'une aiguille.

Le récipient tubulaire 3 est disposé dans le prolongement de la seringue 2, qui sert de moyen de manipulation pour celui-ci. Dans l'exemple de réalisation montré sur les figures, le récipient 3 présente un diamètre sensiblement plus petit que la seringue 2.

7 0107578

Le récipient 3 comporte, du côté de la seringue 2, par exemple en retrait de son extrémité emboîtée sur celle-ci, une cloison transversale interne 8 percée d'un orifice traversant 9. Ainsi, la cloison 8 sépare le volume interne du récipient 3 en deux cavités 3a et 3b en communication par l'orifice 9.

La cavité 3b, plus petite que la cavité 3a, est disposée entre la cloison 8 et la seringue 2, la cavité 3a étant placée entre ladite cloison 8 et un capuchon amovible 10, emboîtable sur l'extrémité du récipient 3, opposée à la seringue 2. De façon connue, une aiguille de prélèvement 11, destinée par exemple à pénétrer dans une veine d'un patient, est emboîtée sur le capuchon 10.

A l'intérieur de la cavité 3a est prévu un piston libre 12, par exemple constitué d'un bloc cylindrique d'élastomère, la paroi de la cavité 3a étant susceptible de former cylindre pour le piston 12.

Compte tenu du piston 12, le volume de la cavité 3a correspond au volume de sang nécessaire pour effectuer l'analyse envisagée.

La conception des différents éléments du système selon l'invention, solidarisés de manière amovible, autorise une grande souplesse d'utilisation et d'interchangeabilité.

Dans un premier temps, l'opérateur emboîte le récipient 3 sur la seringue 2 et pourvoit ledit récipient 3 de son capuchon 10 et de l'aiguille de prélèvement 11.

8

0107578

Le piston 5 est poussé à fond dans la seringue 2, de sorte que le piston 12 est repoussé à l'extrémité de la cavité 3a, proche du capuchon 10 (position montrée sur la figure). Puis, après introduction de l'aiguille 11 dans une veine du patient, le piston 5 est soumis à une traction par l'intermédiaire de la tige 7 et il aspire l'air des cavités 3a et 3b. Il en résulte que le piston libre 12 se déplace vers la cloison 8 en aspirant le sang dans la cavité 3a. Le piston libre 12 sert de joint et le sang ne peut pénérer dans la cavité 3b à travers l'orifice 9. En fin d'aspiration, le piston libre 12 occupe la position 12' et obture l'orifice 9. La cavité 3a est alors pleine de sang et le récipient 3 est désolidarisé de la seringue 2 afin de permettre la réalisation des tests et analyses nécessaires.

Après désolidarisation du récipient 3 de la seringue 2, du capuchon 10 et de l'aiguille 11, ce récipient est apte à subir les manipulations nécessaires aux analyses et tests auxquels son contenu sanguin doit être soumis. En effet, lorsqu'il est disposé verticalement de façon que sa cavité 3a se trouve au-dessus de sa cavité 3b, le récipient 3 forme un simple tube dont le fond est formé par la cloison 8 et le piston 12.

Il est avantageux que la position extrême du bouchon 12 vers l'organe de prélèvement 11 soit limitée par une butée (non représentée), par exemple solidaire du capuchon 10, pour maintenir ledit bouchon en retrait de l'extrémité correspondante du tube. Ainsi, il est possible d'aspirer un volume de sang déterminé dans la cavité 3a, ce volume étant légèrement inférieur à la capacité maximale de ladite cavité, de sorte qu'il n'y a aucun risque de débordement.

REVENDICATIONS


1- Système de prélèvement de liquide physiologique, tel que du sang, comportant des moyens d'aspiration (2) et au moins un récipient (3) susceptible d'être relié, par une de ses extrémités, de manière étanche et amovible auxdits moyens d'aspiration (2), ledit récipient (3) comportant une cloison transversale (8), disposée au voisinage de ladite extrémité reliée aux moyens d'aspiration (2) ou obturant cette extrémité et percée d'un orifice (9), tandis qu'un organe de prélèvement (11) dudit liquide est relié de façon amovible à l'autre extrémité du récipient (3), opposée aux moyens d'aspiration (2), caractérisé en ce que la partie (3a) dudit récipient (3) comprise entre la cloison (8) et l'extrémité reliée audit organe de prélèvement (11) est conformée en cylindre pour un bouchon glissant (12), disposé dans ladite partie (3a) et formant piston libre à l'intérieur de celle-ci.

2- Système de prélèvement selon la revendication 1, dans lequel lesdits moyens d'aspiration (2) sont constitués par une seringue, caractérisé en ce que ledit récipient (3) est emboitable sur ladite seringue sans l'intermédiaire d'une aiguille.

3- Système de prélèvement selon la revendication 2, caractérisé en ce que le volume de la partie (3a) du récipient (3) comprise entre la cloison (8) et l'extrémité à laquelle est rapporté ledit organe de prélèvement est inférieur au volume intérieur du cylindre (6) de la seringue (2).

0107578

4- Système de prélèvement selon l'une quelconque des revendications 1 à 3, caractérisé en ce que le bouchon glissant (12) est formé par un bloc d'élastomère.